# EUROPEAN PATENT APPLICATION

(11) **EP 1 837 040 A2**
(43) Date of publication of application: **26.09.2007**
(21) Application number: 07013908.4
(22) Date of filing: 25.04.2002
(51) Int. Cl.: A61L 27/54, A61L 27/22, A61L 24/00, A61L 24/10, A61L 31/04, A61L 31/16

(54) **Drug delivery matrices to enhance wound healing**

(30) Priority: 25.04.2001 US 286307 P
(62) Divisional of application: 02737999.9
(71) Applicant: Eidgenössische Technische Hochschule Zürich, 8092 Zürich (CH); Universität Zürich, 8006 Zürich (CH)
(72) Inventor: Schmoekel, Hugo, 8046 Zürich (CH); Weber, Franz, 8006 Zürich (CH); Schense, Jason C., 8008 Zürich (CH); Hubbell, Jeffrey A., 1015 Lausanne (CH)
(74) Representative: Klunker . Schmitt-Nilson . Hirsch

(57) **Abstract**

The invention provides a more efficient entrapment of bioactive molecules within a matrix for the controlled delivery of these compounds for therapeutic healing applications. The matrix may be formed of natural or synthetic compounds. The primary method of entrapment of the bioactive molecule is through precipitation of the bioactive molecule during gelation of the matrix, either *in vitro* or *in vivo.* The bioactive molecule is deglycosylated to reduce its effective solubility in the matrix to retain it more effectively within the matrix. Preferably the bioactive factors are deglycosylated members of the cystine knot growth factor superfamily, and particular within the TGFβ superfamily.

## Description

### BACKGROUND OF THE INVENTION

This invention is generally in the field of drug delivery and more specifically in the area of fibrin and synthetic matrices to enhance wound healing.

Fibrin matrices are present naturally in the body and serve as the initial matrix for wound healing. When an injury occurs to tissue, blood vessels are compromised, allowing the precursor molecule, fibrinogen, to invade the wound. The fibrinogen is then enzymatically cleaved and self-catalyzed into a loosely formed gel. The gel is then covalently crosslinked through the action of the transglutaminase, factor XIIIa, resulting in a stable matrix. Pisano, Finlayson and Peyton, Science, 160, 892-893 (1968).

*In vivo,* the final fibrin matrix includes various proteins in addition to fibrinogen, such as serum proteins present during the coagulation process, for example fibronectin and α2-plasmin inhibitor. Factor XIIIa can covalently crosslink these serum proteins to the fibrin matrix, which can then add additional bioactivities to the matrix that can modify the ability of cells to infiltrate and degrade the matrix. Tamaki and Aoki, J Biol Chem, 257, 14767-14772 (1982). These matrices also contain many blood cells, which become entrapped inside the matrix during coagulation, further modifying the biochemical character of the matrix. One major cell type is the platelet, a cell rich with natural supplies of potentially therapeutic growth factors.

One key advantage of fibrin is that it is a matrix that is strongly conductive for cells, allowing them to easily infiltrate the wound site. The process employed involves two key features. First, the matrix contains adhesion sites, allowing the cells to attach and migrate into the gel. Additionally, the matrix is responsive to cell-derived proteolytic activity. This allows the matrix to be degraded locally, allowing the cells to migrate into the matrix uninhibited but preventing global degradation of the matrix. Herbert, Bittner and Hubbell, J Compar Neuro, 365, 380-391 (1996); Pittman and Buettner, Dev Neuro, 11, 361-375 (1989). Therefore, the natural matrix remains at the site of injury until it is infiltrated by cells, at which time it is degraded during this process leading to regenerated tissue.

The natural healing process is sometimes inadequate, such as when this general healing response fails to lead to regeneration of functional specialized tissue. See for example, Robello GT and Aron DN, Semin Vet Med Surg (Small Anim), 7, 98-104 (1992). Therefore, there is a need for a means to induce formation of complete, functional regenerated tissue, especially regenerated specialized tissue.

Many bioactive molecules, including growth factors, peptides, and other assorted molecules, have been discovered which can affect tissue regeneration. Schense and Hubbell, Bioconj Chem, 10, 75-81 (1999). Previous work has shown that growth factors can be precipitated within a fibrin matrix. MacPhee, Druhan *et al*.,76 (1995); U.S. Patent Nos. 6,117,425 and 6,197,325 to MacFee, et al. However, these investigators have not recognized the strong advantages of working with non-glycosylated growth factors, and especially non-glycosylated members of the cystein knot growth, factor superfamily, in particular of the TGFβ superfamily.

Growth factors play an important role in wound healing, and are often naturally present at the site of injury. However if growth factors are applied to the body in high concentrations, adverse effects are likely to be observed. For example if the retention mechanism of BMP in a matrix is not optimized, i.e. if the BMP simply diffuses from the matrix within the first hours, high doses of BMP in the matrix are necessary to cause a local response at the site of injury. As a result most of the BMP circulates freely in the body and ectopic bone formation may occur. It is therefore necessary to keep the freely circulating concentration of the growth factor as low as possible but locally sufficiently high that the desired therapeutic response is triggered at the site of injury. It is known, that for example some growth factor receptors must be occupied for at least 12 hours to produce a maximal biologically effect. A prolonged contact caused by a little but constant stream of growth factor near the site of need therefore is very favorable for a healing response. At the same constant release rate, the release from the matrix is the longer, the higher the initial concentration of retained growth factor in the matrix is.

Therefore it is an object of the present invention to increase the retainable concentration of bioactive molecules, in particular growth factors, in a matrix.

A further object is to provide a method to decrease the solubility of a growth factor in a matrix made from fibrin or made from synthetic polymers.

In particular it is solved by deglycosylated bioactive molecules, in particular by deglycosylated members of the cystine knot growth factor superfamily, in particular by deglycosylated members of the TGF β superfamily.

It is still a further object of the invention to provide compositions and methods for making compositions to improve wound healing., especially by delivery

These objects are solved by the features of the independent claims.

In particular they are solved by delivery of deglycosylated members of the cystine knot growth factor superfamily, in particular non-glycosylated members of the TGFβ superfamily.

### BRIEF SUMMARY OF THE INVENTION

Bioactive molecules are entrapped within a matrix for the controlled delivery of these compounds for therapeutic healing applications. The matrix may be formed of natural or synthetic compounds. The primary method of entrapment of the bioactive molecule is through precipitation of the bioactive molecule during gelation of the matrix, either *in vitro* or *in vivo*. The bioactive molecule may be modified to reduce its effective solubility in the matrix to retain it more effectively within the matrix, such as through the deglycosylation of members within the cystine knot growth factor superfamily and particular within the TGFβ superfamily. The matrix may be modified to include sites with binding affinity for different bioactive molecules, for example, for heparin binding. When these different bioactive molecules are added to the matrix, the bioactive molecules are bound to the matrix both by precipitation within the matrix and by binding to the sites in the matrix, thereby providing enhanced controlled delivery to a patient.

### BRIEF DESCRIPTION OF THE DRAWINGS

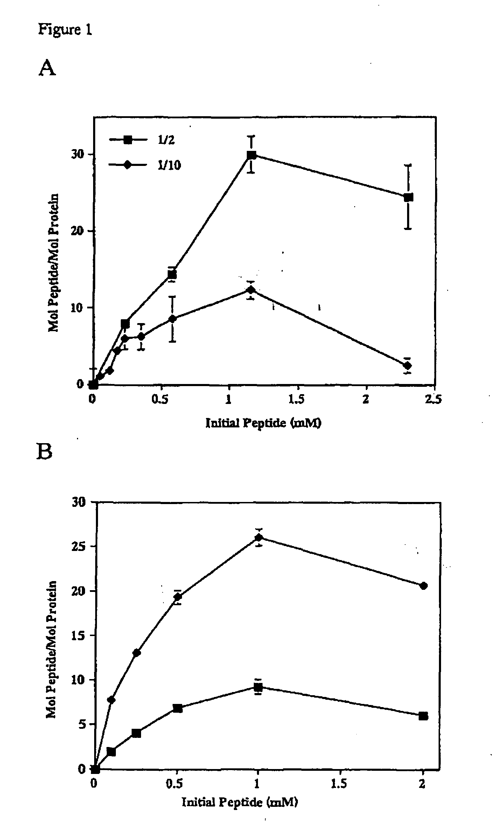

Figures 1A and 1B are graphs measuring the incorporation of a factor XIIIa substrate peptide into fibrin. Fibrin gels were synthesized at 8 mg/mL from pre-diluted Tissucol^{™} Kits (Baxter) (Figure 1A) which were diluted by a factor of 2 (■) and 10 (◆) or from purified fibrinogen either with (◆) or without (■) 1 U/mL of exogenous factor XIIIa added to the prepolymerization mixture (Figure 1B).

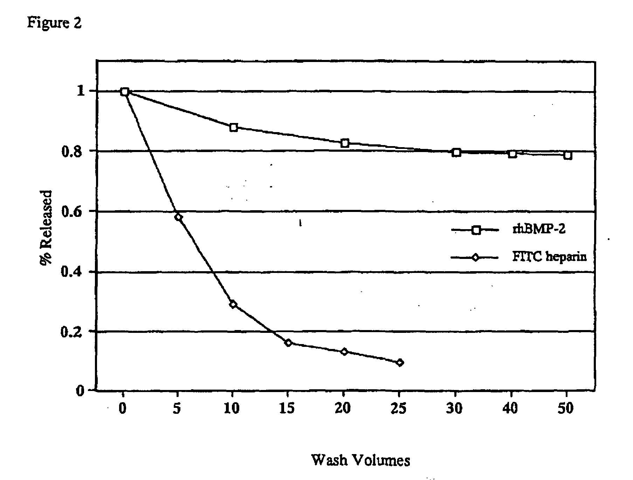

Figure 2 is a graph measuring the retention of bioactive molecules in a fibrin matrix after washing. Two separate bioactive molecules, one water soluble molecule, heparin(◇), and one with low solubility at physiological pH, recombinant human bone morphogenetic protein (rh-BMP-2) (□), were added to the fibrin during polymerization and were repeatedly washed in phosphate buffered saline (PBS).

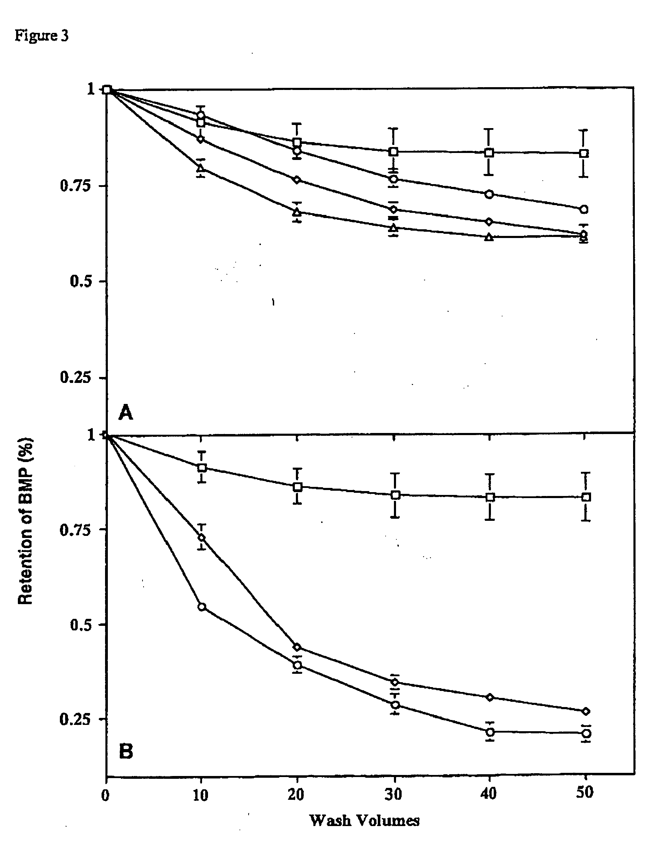

Figures 3A and 3B show the retention of rh-BMP-2 in fibrin gels. In Figure 3A, the fibrin gels were polymerized with 10 (A), 20 (□), 100 (◇) and 200 (O) µg/mL of nonglycosylated rh-BMP-2 present during polymerization of the gel and the percent of rh-BMP-2 remaining in the gel was determined after 10, 20, 30, 40 and 50 wash volumes in PBS. In Figure 3B, the retention in fibrin gels with 20 µg/mL of prokaryotic rh-BMP-2 (□), 20 mg/mL of glycosylated rh-BMP-2 derived from CHO cells (◇) and 20 µg/mL of rh-BMP-2 premixed with equimolar heparin (O) was analyzed as well. Mean values and standard deviations are shown in each figure.

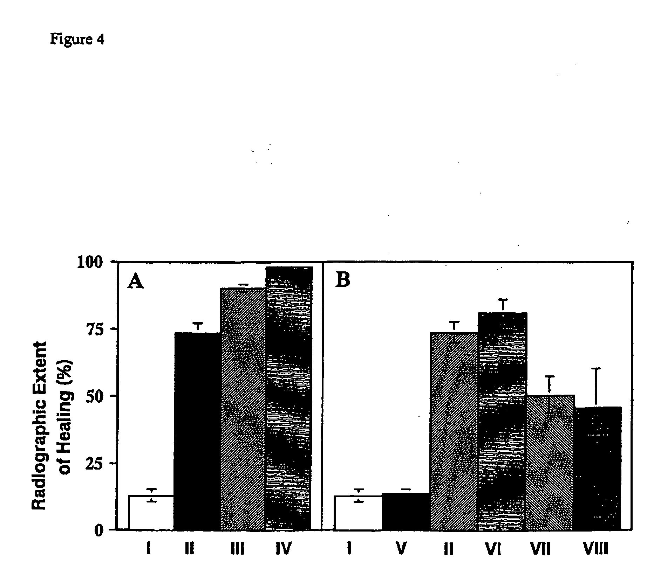

Figures 4A and 4B show healing levels of critical size rat calvarial defects. The healing efficacy of fibrin gels with various glycosylated and nonglycosylated rh-BMP-2 formulations mixed within the gel were measured. In Figure 4 A, nonglycosylated rh-BMP-2 was mixed within the fibrin gel in concentrations of 0 (I), 1 (II). 5 (III), and 20 (IV) µg/mL. Additionally in Figure 4B, fibrin gel (I), fibrin gel with 1 µg/mL nonglycosylated rh-BMP-2 (II), fibrin gel with 1 µg/mL nonglycosylated rh-BMP-2 premixed with an equimolar amount of heparin (VII), fibrin gel mixed with the same heparin level (V), fibrin gel with 1.0 µg/mL glycosylated rh-BMP-2 (VI) with a transglutaminase domain to covalently link it to the fibrin matrix (as described e.g. in US 6,331,422), and fibrin gel with 1 µg/mL glycosylated rh-BMP-2 (VIII) were tested. The average area of the defect filled with calcified tissue after 21 days of healing with standard deviations is depicted in these figures.

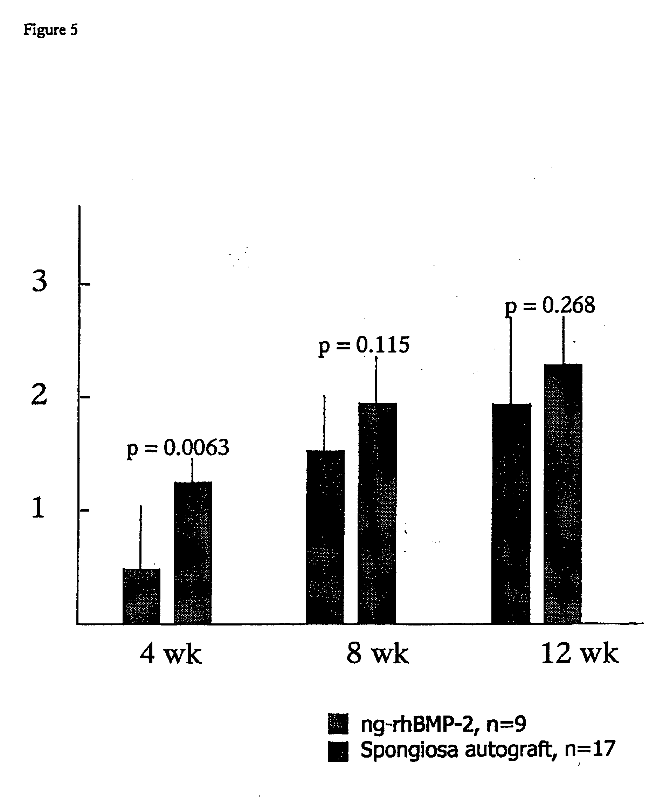

Figure 5 shows the radiologic healing of the canine pancarpal arthrodesis. The efficacy of using nonglycosylated BMP-2 in fibrin matrices was tested in this defect and compared to the clinical standard of cancellous autograft. The mean of the healing score of the three carpal joint rows of nine dogs with a replacement of the autograft with a deglycosylated rhBMP-2/fibrin gel were calculated at four, eight and twelve weeks. A standardized method was developed to determine the extent of healing where 0 corresponded to no mineralization being visible,1 corresponded to some mineralization being visible, 2 corresponded to a defect that is completely mineralized and 3 corresponded to healed and remodeled defects. The deglycosylated rhBMP-2/fibrin treated dogs showed a greater radiologic healing score compared to the control group treated with a autograft.

### DETAILED DESCRIPTION OF THE INVENTION

### I. Compositions

Compositions are formed of a natural or synthetic matrix and a bioactive molecule, in particular a growth factor, which can be administered to a patient to improve wound healing. Compositions may also be understood as precursor materials for the matrix having bioactive molecules entrapped therein, i. e. they may contain at least one component necessary or suitable for forming the matrix with entrapped bioactive molecules. The bioactive compound is released in a controlled manner from the matrix. The bioactive molecule is a deglycosylated member of the cystine knot growth factor superfamily, preferably a non-glycosylated member of the TGFβ superfamily.

In the context of the present invention a bioactive factor is precipitated if the concentration of bioactive factor exceeds the concentration limit that is soluble in the respective vehicle at a predefined pH and temperature . If this definition is met precipitation also can encompass retention due to any physical interaction of the bioactive molecule and the matrix, i.e. adsorption, electrostatic forces, affinity precipitation, co-precipitation etc. The terms entrapment, inclusion and precipitation are used synonymously throughout the application as ways to achieve retention.

"Matrix" shall mean a three dimensional network which can act as scaffold for cell ingrowth and for bioactive molecules over a certain period of time.

"Deglycosylated" bioactive molecules" means bioactive molecules which when found in nature are glycosylated at one or more sites of the molecule however where glycosylation has been removed from the molecule by chemical or enzymatic methods or by producing it as a not-glycosylated molecule. A "deglycosylated growth factor" is a growth factor that can be glycosylated when expressed in a eukaryotic cell and where the polysaccaride sequence or glycosaminoglycans has been either clipped off afterwards or the method of expression is such that the growth factor is not glycosylated. The latter happens for example if the growth factor is expressed in a prokaryotic cell. The terms "deglycosylated", "non-glycosylated" and "not glycosylated" are used synonymously throughout the application.

"Retention" shall mean that at least 10% of the initially applied concentration of bioactive molecule; preferably at least 60% and even more preferably at least 80% is still present in the matrix after 10 wash volumes. Ten wash volume being defined as keeping the matrix in which the bioactive molecule is entrapped in a volume ratio of 1 part matrix to 10 parts of phosphate buffered saline (PBS 0.01M; pH 7.4) for at least 12 hours at 37°C. Retention can be achieved for example by precipitation of the bioactive molecule. "Retainable concentration" shall mean that percentage of the initial concentration which is retained according to the definition given hereinbefore.

The terms "release in a controlled manner" or "controlled release" and "prolonged release" shall have the same meaning and shall express the result of retention. Controlled release is not only due to slow and steady disintegration of the growth factor and subsequent diffusion from the matrix but also due to disintegration and enzymatic cleavage of the matrix.

"Gelation" shall mean the formation of a three-dimensional network and thus the transition from a liquid composition to a viscous composition. The terms "gel" and "matrix" is used synonymously throughout the application. An *in situ* formation of the gel or matrix is understood as being the transition from a liquid to a solid state at the site of application in the body. "Hydrogel" means a class of polymeric material which are extensively swollen in a aqueous medium, but which do not dissolve in water.

"Michael addition or Michael type addition reaction" is the 1,4 addition reaction of a nucleophile on a conjugate unsaturated system under basic conditions. The addition mechanism can be purely polar, or proceed through a radical-like intermediate state(s); Lewis bases or appropriately designed hydrogen bonding species can act as catalysts. The term conjugation can refer both to alternation of carbon-carbon, carbon-heteroatom or heteroatom-heteroatom multiple bonds with single bonds, or to the linking of a functional group to a macromolecule, such as a synthetic polymer or a protein. Double bonds spaced by a CH or CH₂ unit are referred to as homoconjugated double bonds. Michael-type addition to conjugated unsaturated groups can take place in substantially quantitative yields at physiological temperatures, in particular at body temperature but also at lower and higher temperatures than that. They take place in mild conditions with a wide variety of nucleophiles, like amines and thiols. The reaction as used for the present invention is preferably self selective which means that the first precursor component of the reaction reacts much faster with the second precursor component of the reaction than with other compounds present in the mixture at the site of the reaction, and the second precursor component reacts much faster with the first precursor component than with other compounds present in the mixture at the site of the reaction. As used herein, a nucleophile preferentially binds to a conjugated unsaturation, rather than to other biological compounds, and a conjugated unsaturated group preferentially binds to a nucleophile rather than to other biological compounds.

"Polymeric network" means the product of a process in which substantially all of the monomers, oligo- or polymers are bound by intermolecular covalent linkages through their available functional groups to result in one huge molecule.

"In situ formation" refers to the ability of mixtures of precursor components which are substantially not crosslinked prior to and at the time of injection to form covalent linkages with each other at a physiological temperature at the site of injection in the body

As used herein, the words "polymerization" and "cross-linking" are used to indicate a linking of multiple precursor component molecules to result in a substantial increase in molecular weight. "Cross-linking" further indicates branching, typically to yield a polymer network.

By "functionalize" is meant to modify in a manner that results in the attachment of a functional group or moiety. For example, a molecule may be functionalized by the introduction of a molecule which makes the molecule a strong nucleophile or a conjugated unsaturation. Preferably a molecule, for example PEG, is functionalized to become a thiol, amine, acrylate, or quinone.

By "functionality" is meant the number of reactive sites on a molecule. As used herein, the functionality of a strong nucleophile and a conjugated unsaturation will each be at least two. Mixing two components, for example, a strong nucleophile and a conjugated unsaturation, with functionalities of two each will result in a linear polymeric biomaterial, and the mixing to two components with functionalities of at least two each, one of the components having a functionality of more than two, will result in a crosslinked biomaterial.

As used herein, by "regenerate" is meant to grow back a portion, or all of, a tissue. For example, the present invention features methods of regenerating bone following trauma, tumor removal, or spinal fusion, or for regenerating skin to aid in the healing of diabetic foot ulcers, pressure sores, and venous insufficiency. Other tissues which may be regenerated include, but are not limited to skin, bone, nerve, blood vessel, and cartilage tissue.

As used herein "peptide" and "protein" are differentiated by their chain length according to chain length definitions usual in the art. Preferably "Peptide" shall mean polyaminoacids up to 30 amino acids, most preferably from about 10 to 20 amino acids, whereas proteins are preferably polyaminoacids above 30 amino acids.

### A. Matrix

The matrices may be biodegradable or nondegradable. The matrices may be made of synthetic or natural polymers, oligomers and monomers. The terms polymer, oligomer and monomer are used in the usual sense of the word. Synthetic polymers, oligomers and monomers include those derived from polyalkyleneoxide precursor molecules, such as poly(ethylene oxide) (PEO), poly(ethylene glycol) (PEG) and copolymers with poly(propylene oxide) (PEG-co-PPG), poly(vinyl alcohol) (PVA), poly(vinylpyrrolidone) (PVP), poly(ethyloxazoline) (PEOX), polyaminoacids, and pseudopolyamino acids, and copolymers of these polymers. Sawhney AS, Pathak CP and Hubbell JA, Macromolecules, 26, 581-587 (1993). Copolymers may also be formed with other water-soluble polymers or water insoluble polymers, provided that the conjugate is water soluble. An example of a water-soluble conjugate is a block copolymer of polyethylene glycol and polypropylene oxide, commercially available as a Pluronic^{™} surfactant (BASF).

Natural polymers, oligomers and monomers include proteins, such as fibrinogen, fibrin, gelatin, collagen, elastin, zein, and albumin, whether produced from natural or recombinant sources, and polysaccharides, such as agarose, alginate, hyaluronic acid, chondroitin sulfate, dextran, dextran sulfate, heparin, heparin sulfate, heparan sulfate, chitosan, gellan gum, xanthan gum, guar gum, water soluble cellulose derivatives, and carrageen. These polymers are merely exemplary of the types of matrices that can be utilized and are not intended to represent all the matrices within which entrapment is possible.

### Fibrin matrices

Due to its natural role in healing and cell infiltration conductive abilities, fibrin is a preferred choice for making a matrix. In a preferred embodiment, the matrix is a fibrin gel, created from any source of fibrinogen. When mixed with the proper amount of thrombin, calcium and bioactive molecule, a fibrin gel can be created at physiological conditions which means at conditions as they can be found in human and animal beings. However fibrin gel formation can also occur outside the body and , in the presence of thrombin and calcium, mainly depends on temperature and pH. The fibrin gel can be formed outside the body at a temperature range of between 25°C to 40°C and a pH range of between 7 to 8. If the bioactive molecule is not soluble at these conditions, it will precipitate during polymerization and become entrapped within the matrix.

As an additional attribute, many forms of fibrin are available for use as a matrix. Fibrin gels can be synthesized from autologous plasma, cryoprecipitated plasma (e.g. fibrin glue kits, which are available commercially), fibrinogen purified from plasma, and recombinant fibrinogen and factor XIIIa. Each of these materials provides a fundamentally similar matrix, with small variations in the biochemical compositions. Sierra DH, J Biomater Appl, 7, 309-352 (1993). Similarities between these materials exist both in specific enzymatic bioactivity and general healing responses.

### Synthetic matrices

Synthetic matrices are known in tissue regeneration and wound healing. These include macroporous sponges of degradable polymers such as polylactic acid and its copolymers as well as hydrogel matrices based on water-soluble polymers such as PEG. In one preferred formulation, PEG is used as a base precursor material to obtain an enzymatically degradable matrix. PEG is functionalized with chemically reactive groups, such as acceptor groups in the form of conjugated unsaturated bonds for Michael-type addition reactions, including acrylates, vinyl sulfones and acrylamides. Preferably the PEG is a four arm PEG of an average weight molecular weight of between 15 to 25000 kD. These precursors (in solution) are mixed with peptides as a second precursor component (in solution) that contain two or more reduced cysteine residues (nucleophilic thiol groups), with protease substrate sites intervening between these cysteine sites. Under basic conditions a gel rapidly forms by a Michael-type addition reaction between the multi-thiol component (the functionalized peptide) and the multi-acceptor component, (the functionalized PEG) so long as the sum of the functionality of the multiacceptor (number of Michael acceptor groups (m) per molecule) and the functionality of the multithiol (number of thiol groups (n) per molecule) is greater than 5. The Michael addition between the thiol and the acceptor groups works from pH 6.5 up to very basic conditions at a wide variety of temperature. But when the precursor components are injected in the body for an in-situ formation of the matrix the pH must be appropriate for the body and therefore in a preferred embodiment the pH is between 7 and 8. Preferred temperature range is between 25°C to 40°C when the gel is formed outside the body. Inside the body the gel is formed at body temperature. When the peptide is designed to be a substrate for plasmin or a matrix metalloproteinase, the resulting synthetic gels degrade in response to the enzymatic matrix remodeling influence of cells. The multithiol, i.e. the nucleophilic precursor component does not necessarily have to be a peptide. If for example the matrix does not have to be enzymatically degradable, the nucleophilic precursor component, i.e. the multithiol can be a PEG as well. The gel can further comprise cell attachment sites, like for example RGD sequences, covalently bound to the matrix to help ingrowth and attachment of cells into the matrix. The cell attachment site can be bound to the matrix by Michael addition reaction, too. For that the RGD is modified such that it contain free thiol/cysteine groups for reaction with the conjugated unsaturated bond.

### B. Bioactive molecules

The matrices can be further modified by including bioactive molecules, often derived from development, to enhance the regeneration of the wounded tissue. Pandit et al., J Biomater Appl, 14, 229-42 (2000); Hildebrand et. al., Am J Sports Med, 26, 549-54 (1998); Quirinia A, Scand J Plast Reconstr Surg Hand Surg, 32, 9-18 (1998).

The type of molecule that is entrapped can be one from a large list of possible bioactive molecules, including growth factors, peptides, enzymes, protease inhibitors, antibiotics, synthetic homologues and other assorted molecules. Preferred bioactive molecules have reduced solubility at physiological pHs.

### Growth Factors

Growth factors are particularly useful because they provide a well-characterized chemical entity that has been shown to play an important role in wound healing, and are often naturally present at the site of injury. Additionally, growth factors are pluripotent molecules, allowing them to activate many different cell types and induce a complicated healing response.

The crystal structure of members of the cystine knot growth factor superfamily have been reported as having unusual folds, involving intramolecular disulphide bridges. In transforming growth factor-beta 2, platelet derived growth factor (PDGF), nerve growth factor (NGF) and human chorionic ganodotropin (hCG), six conserved cysteines (I to IV in sequence order) form three disulphide links arranged in a knot-like topology. Cysteines [II to V] and [III to VI] form a ring of eight amino acids through which the remaining disulphide bond (Cys[Ito IV]) penetrates. This topology differs from the structural class of inhibitor like cysine knots in which Cys[III-IV] penetrates a macrocyclic ring formed by CYS[I-IV]and Cys[II-V]. Thus cystine knots fall into two structural classes: growth factor type and inhibitor-like cystine knots. Members of the cystine knot growth factor superfamily is the platelet derived growth factor (PDGF) superfamily, the transforming growth factor beta (TGF β) superfamily and the glycoproteins alpha family. Examples of individual growth factors are BMP's, PDGFs, TGF betas. Not all of the growth factors are glycosylated when expressed by eukaryotic cells, the TGF beta 1,2 and 3 for example are never glycosylated irrespective of the expression system used.

Within the TGF beta superfamily the most common molecules used for bone regeneration come from the bone morphogenetic protein (BMP) family. Initially, BMPs were used as a cocktail of growth factors purified from bone. Urist et al., Proc Natl Acad Sci U.S.A., 76,1828-32 (1979). These mixtures were entrapped within a fibrin matrix and their therapeutic efficacy was measured. This provided an interesting preview of the therapeutic potential of fibrin mixed with BMP. However, the effects of each of the various growth factors present in the matrix were not determined.

BMP-2 and BMP-7 (OP-1), both have heparin binding affinity, are soluble at low pHs and are strong inducers of bone healing. Wozney JM, Prog Growth Factor Res, 1, 267-80 (1989); Wozney et al., J Cell Sci Suppl, 13, 149-56 (1990). rh-BMP-2 has demonstrated the greatest healing potential and is even able to induce bone formation at an ectopic site. Jin et. al., J Biomed Mat Res, 52, 841 (2000). Since the solubility of rh-BMP-2 at physiological conditions is low, it can precipitate within a matrix. Thus, this molecule fits the characteristics necessary for delivery.

The precipitation of this growth factor and thus its prolonged release has been further improved by using a recombinant form of rh-BMP-2, which is not glycosylated, and therefore is less soluble in fibrin or synthetic matrices. It is also possible to improve the precipitation of this growth factor when it is expressed in a glycosylated form by chemically or enzymatically deglycosylating it. The structural homology between the members of the BMP family is high; therefore, the results obtained with rh-BMP-2 can be expected to be obtained with the other BMPs, including BMP-7 (OP-1).

### TGFβ superfamily

The BMPs are themselves members of the transforming growth factor beta (TGFβ) superfamily, and the structural homology between the members of the TGFβ superfamily is also high. As such, results obtained with BMP-2 can be expected to be obtained with other members of the TGFβ superfamily and members of the cystine knot growth factor superfamily. The precipitation of growth factors that are members of the TGFβ superfamily and their prolonged release is further improved by using recombinant forms that are not glycosylated and are therefore less soluble.

### Deglylosylated BMPs

Deglycosylated versions of BMP and other growth factors can be obtained using a number of techniques. Several methods of deglycosylation are available in common practice, both chemical and biological. One chemical method occurs through the use of hydrogen fluoride. Briefly, glycosylated proteins are mixed with polyhydrogen fluoride, pyridine and a scavanger. This leads to essentially complete deglycosylation without modification of the protein itself. Biological methods center around the use of enzymes to cleave the glycosaminoglycans from the protein or expression in bacteria. Two examples are N-glycanase (Un, Zhang et al. J Neurochem,63, 758-768 (1994)) or glycopeptidase F (Chen and Gonatas Biochem Biophys Res Commun,234,68-72 (1997)), which can be used to deglycosylate proteins. These examples are merely illustrative of the biological and chemical methods that can be used to create a deglycosylated protein from an eukaryotic source (i.e. glycosylated), and are not a complete list of all possible methods. Using these standard techniques, the solubility of the glycosylated rh-BMP-2 can be made to mimic that of nonglycosylated rh-BMP-2. Furthermore, one can use excipients to reduce the solubility of proteins, e.g. polymers of opposite charge to reduce the net charge of the protein.

### II. Methods of Incorporating Bioactive Molecules within the Matrix

Two primary methods for delivering bioactive factors are through biochemical and physical methods. In biochemical methods, matrices are created which have a chemical affinity for the bioactive factor of interest. When the matrix is mixed with the bioactive molecule, the release of the molecule can be delayed or eliminated. Physical methods, which may be used to retain the bioactive molecules in the matrix, include precipitation, co-precipitation, affinity precipitation, and physical entrapment. For example, in one embodiment, the bioactive molecules are precipitated inside a fibrin matrix to improve retention. This matrix, which contains the precipitated molecules, has a significant potential for wound healing.

### A. Precipitation and chemical modification of matrix

Precipitation can be combined with other retention methods to produce biomaterials which improve wound healing. One example is the use of modified biomaterials, which contain sites with binding affinity for bioactive molecules. The bioactive molecule is bound to the matrix, enhancing retention of the bioactive molecule in the matrix. For example, a matrix can be modified to include binding sites with heparin affinity and heparin can be added to bind with the matrix. Then, if the bioactive molecule has heparin binding affinity, the bioactive molecule will bind with the heparin and thus be retained in the matrix. This method can be performed in conjunction with the use of precipitation for slower release kinetics.

In one embodiment, the retention of rh-BMP-2 is enhanced by binding to heparin which is bound to a modified fibrin matrix. Thus deglycosylated rh-BMP-2 is retained in the matrix because it is precipitated within the matrix due to its poor solubility and bound to heparin due to its heparin binding affinity.

### III. Methods of Using the Matrices to Enhance Wound Healing

### A. Types of Patients in need of Composition

These matrices provide a wide range of patients with therapies for healing bony defects. In one embodiment, the modified fibrin or synthetic matrices serve as a replacement for bone grafts, and thus may be applied in many of the same indications. These indications include, but are not limited to, spinal fusion cages, healing of non- union defects, bone augmentation, and dental regeneration. Additionally, in another embodiment, these matrices can be used in implant integration. In implant integration, implants can be coated with a modified matrix, either natural or synthetic, inducing the neighboring bone area to grow into the surface of the implant and preventing loosening and other associated problems. These examples are merely illustrative and do not limit the number of possible indications for which the matrices described herein can be used. In another embodiment growth factor enriched matrices can be used for healing chronic wounds in skin

### B. Methods of Administration

In one embodiment, the material is applied to the wound area as a preformed matrix. In a second embodiment, the material is gelled *in situ* in the body. In both of these embodiments, the matrix material can be made from synthetic or natural precursor components. It goes without saying that, irrespective of the kind of precursor component used, it is to be avoided that the precursor components are combined or come into contact with each other under conditions that allow polymerization of said components prior to application of the mixture to the body. In the overall sense this is for example achieved by a system comprising at least a first and a second composition separated from each other wherein the first and the second composition comprise components that form a three dimensional network upon mixing under conditions that allow polymerization of said components. Additionally the system comprises a biological active molecule being selected from deglycosylated members of the cystine knot growth factor superfamily in at least one of the compositions. Depending on the precursor components and their concentration gelling can occur quasi instantaneously after mixing; which makes injection, i.e. squeezing of the gelled material through the injection needle, almost impossible.

In one embodiment the matrix is formed from fibrinogen. Fibrinogen, through a cascade of various reactions gels to a matrix, when brought in contact with thrombin and a calcium source at appropriate temperature and pH. For storage it is necessary to not allow the three components to come into contact. As long as at least one of the three parts is kept separated any other combination of the three components is feasible. In a first embodiment fibrinogen is dissolved (which may contain additionally aprotinin to increase stability) in a buffer solution at physiological pH (in a range from pH 6.5 to 8.0, preferably from pH 7.0 to 7.5) and stored separately from a solution of thrombin in a calcium chloride buffer (concentration range of from 40 to 50 mM). The buffer solution for the fibrinogen can be a histidine buffer solution at a preferred concentration of 50 mM comprising additionally NaCl at a preferred concentration of 150 mM or TRIS buffer saline (preferably at a concentration of 33mM). The bioactive molecule may be present in either the fibrinogen or the thrombin solution. In a preferred embodiment the fibrinogen solutions contains the bioactive molecule. The fibrinogen and the thrombin solutions can additionally be stored frozen to enhance storage stability. Prior to use the fibrinogen part and the thrombin part are defrosted (when necessary) and mixed. In another system fibrinogen and thrombin can be stored separated from the calcuium source. In still another embodiment the fibrinogen can be stored with the calcium source separated from the thrombin.

In another preferred embodiment both fibrinogen and thrombin are separately stored in lyophilised form. Either of the two can contain the bioactive molecule. Prior to use the tris or histidine buffer is added to the fibrinogen, the buffer may additionally contain aprotinin. The lyophilized thrombin is dissolved in the calcium chloride solution. Subsequently the fibrinogen and the thrombin solutions are mixed, again preferably by way of combining the containers/vials/syringe bodies comprising the solutions by a two way connecting device having a needle attached at one of its sides. It is very convenient if the vials are bipartited thus having two chambers separated by an adjustable partition rectangular to the syringe body wall. One of the chambers can contain lyophilised fibrinogen, the other chamber contains an appropriate buffer solution. If pressure is applied to one end of the syringe body, the partition moves and releases bulges in the syringe wall in order that the buffer can float into the fibrinogen chamber and dissolve the fibrinogen. A bipartite syringe body is used for storage and dissolution of the thrombin in the same way. If both fibrinogen and thrombin are dissolved, both bipartite syringe bodies are attached to the two way connecting device and the contents are mixed by squeezing them through the injection needle attached to the connecting device. The connecting device additionally can comprise a static mixer to improve mixing of the contents.

In a preferred embodiment the fibrinogen is diluted eight fold and thrombin is diluted 20 fold prior to mixing. This ratio results in a gelation time of approximately one minute.

In another preferred embodiment the matrix is formed from synthetic precursor components capable of undergoing a Michael addition reaction. Since the nucleophilic precursor component (the multithiol) only reacts with the multiacceptor component (the conjugated unsaturated group) at basic pH, the three components which have to be kept separated prior to mixing is the base, the nucleophilic component and the multiacceptor component. Both the multiacceptor and the multithiol component are stored as a solution in a buffer. Both of the compositions can comprise the cell attachment site and additionally the bioactive molecule. Thus, the first composition of the system can for example comprise the solution of the nucleophilic component and the second composition of the system can comprise the solution of the multiacceptor component. Either of the two compositions can comprise the base or the base can be present in both of the compositions. In another embodiment the multiacceptor and the multithiol can be comprised as solution in the first composition and the second composition can comprise the base. Connecting and mixing occurs in the same way as previously described for fibrinogen. In addition the bipartite syringe body is equally suited for the synthetic precursor components. Instead of fibrinogen and thrombin the multiacceptor and multithiol components are stored in pulverized form in one of the chamber and the other chamber containes the basic buffer.

### C. Dosage

The matrices typically contain a dosage of 0.01 to 5 mg/mL of bioactive molecule. This dosage range is in accordance with the levels of active protein used in other clinical trials. However, lower doses may also be used due to the improved delivery that the matrices provide. For example, when non-glycosylated rh-BMP-2 was used in healing non-union cranial defects in rats, very low doses of 1-10 µg/mL were effective. As such, when using precipitated growth factors and especially advantageous forms such as non-glycosylated forms, significant reductions in dosing are possible. Thus less protein is necessary to get the same result.

The delivery time of the bioactive molecule occurs within several weeks of administration. Within two to four weeks, it is likely that the original matrix has been completely remodeled and all of the bioactive molecules have been released.

The present invention can be further understood by reference to the following non-limiting examples.

### Example 1: Determination of Incorporation into Fibrin Gels.

A test measuring the native enzymatic activity of the coagulation enzyme, factor XIIIa, was performed. This test was performed by measuring the ability of fibrin gel from two different sources to covalently incorporate a synthetic substrate during the coagulation process. One source of the fibrin gel came from a fibrin glue kit, while the second source came from a purified fibrin gel. Peptides derived from α2-plasmin inhibitor can be covalently incorporated into fibrin gels through the action of factor XIIIa. Thus, one method for testing the enzymatic activity in a fibrin gel or dilution thereof involves testing the ability of different fibrin sources to incorporate this same peptide. The gels were synthesized with various amounts of fluorescently labeled peptide and washed with PBS (0.03M, pH 7.4) to remove free peptide from the matrix. The gels were then degraded with a minimal amount of plasmin necessary and analyzed with size exclusion chromatography. The amount of fluorescent signal (i.e. peptide) bound to the matrix was determined when various dilutions of fibrin glue kits or purified fibrin gels were employed. This result was correlated to the amount of crosslinking activity present in the matrix.

Figures 1A and 1B depict the results of this test. The results of the test demonstrated that when similar concentrations of fibrin are tested, the level of incorporation is similar. For example, the biochemical enzymatic activity in a fibrin glue kit (Figure 1A) proved to be similar to that in a purified fibrin gel (Figure 1B). However, higher protein (and factor XIIIa) concentrations lead to higher incorporation levels.

### Example 2: In Vivo Comparison of Fibrin Gels from different Sources.

A non-glycosylated recombinant form of a bone morphogenetic protein, which was prepared from prokaryotic cells (*E. coli),* (rh-BMP-2) was mixed in different fibrin gels, and the gels were tested in a rat femur defect. Because the protein was expressed in a prokaryotic system, it was not glycosylated. Fibrin gels were synthesized from a variety of sources. Purified fibrinogen from Sigma Chemical and a blood bank were employed, as well as fibrin glues (Baxter) at several dilutions. These gels were loaded with rh-BMP-2 and placed in a critical size (5mm full thickness) femur defect. It was observed that all of the fibrin glue dilutions employed and the Sigma fibrin gave a similar healing response, leading to bridging of every critical size defect. The fibrin from the blood bank gave a lower overall response, which was more likely due to cell infiltration properties than to retention of the rh-BMP-2. (unpublished data) Therefore, while the healing rate varied, the ability of the various matrices to retain rh-BMP-2 was not dependent on the exact fibrin matrix employed.

### Example 3: Comparison of Retention of Soluble and Insoluble Bioactive Molecules in a Fibrin Matrix.

This *in vitro* assay involved comparing the release kinetics of the entrapped non-glycosylated rh-BMP-2 to the release kinetics of a molecule that is known to have high solubility at physiological pH. Fibrin gels were polymerized using purified fibrinogen (Sigma) at 8 mg/mL and 2 U/mL thrombin at pH 7.4. Calcium was added so that the final concentration was 2.5 mM to increase the rate of gelation

These gels were synthesized with a bioactive molecule present during the coagulation process and the retention of the molecule inside the fibrin matrix was determined. Gels were washed and kept in phosphate buffered saline (PBS 0.01M, pH 7.4) at 37°C and the wash was changed every 12 hours. After thorough washing, the gels were degraded with 0.05 Units of plasmin. The amount of each bioactive molecule present in the washes and in the degraded matrix was determined

In the first test, the retention of FITC-labeled heparin, a highly soluble molecule, was tested. The amount of fluorescence in the washes and in the degraded gels was analyzed via fluorescence spectroscopy, and the percent of heparin released in each wash volume was determined. Fibrin gels contain a natural heparin binding sequence, therefore it was expected that there would be some retention of the heparin within the matrix. The fluorescence spectroscopy revealed that the release of heparin from the matrix was delayed relative to diffusion-controlled release (see Figure 2). It is likely that this delay is due to the heparin binding site in fibrin. However, much of the heparin did diffuse out of the matrix, with essentially all of the heparin released from the matrix (see Figure 2).

In the second test, non-glycosylated rh-BMP-2, a molecule with low solubility at pH 7.4, was trapped inside the fibrin matrix during polymerization. The release profile for rh-BMP-2 demonstrates that rh-BMP-2 was released from the matrix more slowly than FITC-labeled heparin. Further, about 80% of the initial dose remained precipitated inside the matrix at the completion of the test (see Figure 2).

A range of initial non-glycosylated rh-BMP-2 concentrations were tested, from 10 to 200 µg/mL. It can be seen that between 60 and 80% of the rh-BMP-2 remained in the gel even after 50 wash volumes (Fig 3A). There is not a noticeable concentration dependence on the retention of rh-BMP-2, with high levels retained at all the relevant concentrations employed. Clearly then, this precipitation effect works at multiple concentrations of growth factor.

This result is due to the low solubility of the non-glycosylated rh-BMP-2 at pH 7.4, which caused a significant amount of the rh-BMP-2 to precipitate inside the matrix. Thus, a physical mechanism, such as precipitation, can be used to entrap bioactive molecules within fibrin matrices.

In order to test the mechanism for the high retention of nonglycosylated rh-BMP-2, retention of higher soluble species of rh-BMP-2 was studied. One possible method to improve the solubility of rh-BMP-2 is to link it with a highly soluble polysaccharide. This has been demonstrated previously with heparin, where it has been shown that the stability of proteins in solution can be enhanced when they are electrostatically bound to heparin. (Pineda-Lucena, Jimenez et al. J Mol Biol, 64, 162-178 (1996)) Alternatively, the polysaccharide can be covalently bound directly to the protein by using naturally (Rajan, Tsarbopoulos et al. Biochem Biophys Res Commun, 206, 694-702 (1995)) or synthetically (Tams, Vind et al. Biochem Biophys Acta,1432, 214-221 (1999)) glycosylated versions. If the low solubility of rh-BMF-2 is the cause for its high retention, then both of these formulations should have a correspondingly lower retention. When heparin was premixed with rh-BMP-2 in a 1:1 molar ratio, the retention of rh-BMP-2 were demonstrated to be much lower, with only 20% being retained within the fibrin matrix (p<0.05). This was tested further by measuring the release of a glycosylated rh-BMP-2, derived from CHO cells. When the retention of this rh-BMP-2 was assessed, the release was very high, with only 30% remaining within the gel (Figure 3B), an amount that is not statistically different from the result obtained with the mixture of prokaryotic rh-BMP-2 with heparin (Figure 3A). Based on these results, it is likely that the mechanism by which prokaryotic rh-BMP-2 is retained at such high levels is through precipitation in the matrix. Thus, these results demonstrate the advantageous nature of non-glycosylated rh-BMP-2 within matrices, such as fibrin matrices, in the promotion of healing.

The results of Example 3 demonstrated the very advantageous usage of non-glycosylated rh-BMP-2 in bone regeneration in fibrin matrices. Non-glycosylated rh-BMP-2 will likewise be advantageous for regeneration of bone, as well as other tissues, in matrices other than fibrin. Moreover, the results of this Example may be extended by structural similarity to other members of the BMP family, and by the same structural similarity to other members of the TGFβ superfamily, including TGFβ1, TGFβ2, TGFβ3, and the numerous other members of the TGFβ superfamily. Furthermore, these results may also be extended to other wound healing situations, including healing of chronic wounds in the diabetic, in the venous insufficiency patient, and the pressure ulcer. In these and essentially all situations in promotion of healing and regeneration under the stimulatory influence of a growth factor, the prolonged presence of the growth factor in a regeneration matrix is desirable. As such, non-glycosylated members of the TGFβ superfamily are broadly useful in the promotion of wound healing and tissue regeneration.

Examples 4 and 5 describe *in vivo* tests, in which the bioactivity of the precipitated deglycosylated rh-BMP-2 was examined. The *in vivo* assays involved using matrices with entrapped rh-BMP-2 in critical size bony defects in the rat. These defects do not spontaneously heal on their own. Therefore these models allow one to determine the osteogenic potential of a particular treatment since the background healing is very low. Schmitz JP, Clin Orthop 1986, 205, 299-308. Here, both a long bone model (5 mm full segmental femur defect) (Example 4) and a cranial model (8mm defect) (Example 5) were employed. In each model, the healing potential of a fibrin matrix with rh-BMP-2 entrapped was compared to that for a fibrin matrix lacking rh-BMP-2.

### Example 4: In vivo Healing of a Critical Femur Defect in a Rat.

Fibrin gels were polymerized using purified fibrinogen (Sigma) at 8 mg/mL and 2 U/mL thrombin at pH 7.4. Some of the gels included prokaryotic rh-BMP-2 mixed into the solution before gelation. Calcium was added to increase the rate of gelation.

Defects of 5 mm full-thickness were created in a rat femur and filled with fibrin matrices. Some matrices contained deglycosylated rh-BMP-2 while others did not. For the matrices with rh-BMP-2, three different amounts of rh-BMP-2 were tested (2 µg, 5 µg, and 10 µg). The amount of regenerated bone within the defect was measured at four weeks to determine the efficacy of precipitated rh-BMP-2 in bone regeneration and compared to the results of the fibrin gels which lacked rh-BMP-2.

When the fibrin gels lacking rh-BMP-2 were explanted and tested at four weeks, the level of new, calcified bone within the defect margin was very low. Instead, the defect was bridged with fibrous tissue resulting in nonfunctional healing. None of the defects filled with a base matrix demonstrated complete healing.

At four weeks, fibrin gels with either 2, 5 or 10 µg of rh-BMP-2 added to the polymerization mixture were explanted and tested. Every animal that received either 5 or 10 µg of rh-BMP-2 in the defect exhibited complete healing, with the original defect filled with calcified bone and bone marrow and the entire gap bridged with calcified tissue. Animals that received materials with 2 µg of rh-BMP-2 in the defect healed very well as well, with 69% of the original defect area filled with mature, woven bone. The average percentage of bone defect area filled with calcified bone is shown in Table 1. In every sample, there was no sign of inflammation or scarring at the site of healing.

**Table 1: Percent of Calcified Tissue in Healed Femur Defects**

| Treatment | Regenerated Bone (%) |
|---|---|
| Fibrin | 7 |
| Fibrin + 2 µg rh-BMP-2 | 69 |
| Fibrin + 5 µg rh-BMP-2 | 100 |
| Fibrin + 10 µg rh-BMP-2 | 100 |

### Example 5: Healing the In vivo critical Cranial Defect.

### In vivo work with fibrin matrices

Fibrin gels were polymerized using purified fibrinogen (Sigma) at 8 mg/mL and 2 U/mL thrombin at pH 7.4. Some of the gels included prokaryotic rh-BMP-2 mixed into the solution before gelation. Calcium was added to increase the rate of gelation.

Defects of 8 mm were created in rat crania and filled with either a fibrin gel or a fibrin gel with rh-BMP-2 precipitated inside. For the matrices with deglycosylated rh-BMP-2, three different amounts of rh-BMP-2 were tested (1 µg, 5 µg, and 20 µg). The amount of regenerated bone within the defect was measured at three weeks to determine the efficacy of precipitated rh-BNIP-2 in bone regeneration and compared to the results when fibrin gels were synthesized without rh-BMP-2 present.

At three weeks, the fibrin gels lacking rh-BMP-2 were explanted and tested. The level of new, woven bone within the defect margin was very low. The amount of new, woven bone within the defect was measured to be about 13% of the original defect area. None of the matrices led to complete healing of the defect, and most of the defect was still filled with fibrous tissue.

Fibrin gels which contained deglycosylated rh-BMP-2, contained either 1, 5 or 20 µg of rh-BMP-2 added to the polymerization mixture. These materials were explanted and tested at three weeks. All of the defects treated with 20 µg of precipitated rh-BMP-2 were completely filled with woven bone and bone marrow. The defects with 5 µg of rh-BMP-2 (III) had nearly complete healing, with 90% of the original defect area filled with calcified tissue. The defects with 1 µg of rh-BMP-2 (I) showed very good healing as well, with 73% of the defect area filled with new, woven bone. The average amount of the defect area filled with calcified tissue is shown in Table 2 and Figure 4A. From Table 2, a dose dependence response is shown, with higher concentrations of precipitated rh-BMP-2 leading to better healing results. Finally, in every sample, there was no sign of inflammation or scarring at the site of healing or on the dura.

**Table 2: Percent of Calcified Tissue in Healed Cranial Defects**

| Treatment | Regenerated Bone (%) |
|---|---|
| Fibrin | 13 |
| Fibrin + 1 µg rh-BMP-2 | 73 |
| Fibrin + 5 µg rh-BMP-2 | 90 |
| Fibrin + 20 µg rh-BMP-2 | 100 |

The two forms of rh-BMP-2 that had higher solubility were tested as well and these had significantly lower healing responses. When 1 µg of nonglycosylated rh-BMP-2 was premixed with an equimolar amount of heparin (VII) and added before polymerization of a fibrin gel, the level of healing dropped to 50%, statistically lower than the equivalent healing with 1 µg of rh-BMP-2 alone (II ; 73%) (p<0.05). This result cannot be attributed to the effect of heparin alone because fibrin with heparin premixed within the matrix provided a similar healing response to that of fibrin alone (see the first two columns of Figure 4B (I and V). Similarly, when glycosylated rh-BMP-2 was used, a lower healing response was achieved (VIII). Glycosylated rh-BMP-2 has a much higher specific activity than non-glycosylated rh-BMP-2, due to better folding, better dimerization and many other factors. However, when an equivalent molar dose (1 µg) of glycosylated rh-BMP-2 was employed, the healing response was only 44% of the defect filled with calcified tissue, in comparison to the 73% result obtained with nonglycosylated rh-BMP-2 (Figure 4B).

As expected, using a plain fibrin matrix in the absence of any bioactive molecules to heal a critical size defect led to a very poor healing response, with little calcified tissue in either the femur or cranial model. This very low background healing with the control matrix demonstrates that the strong healing response that results when rh-BMP-2 is precipitated within the matrix represents a strong therapeutic healing ability in bony tissue. Therefore, physical processes, such as precipitation, especially through the use of a non-glycosylated TGFβ superfamily growth factor, provide a key tool for developing therapeutic matrices for wound healing.

### In vivo work with synthetic matrices

Enzymatically degradable synthetic matrices were tested in the same cranial defect model as the fibrin matrices described above. The synthetic gels were formed by reacting a four arm PEG-vinylsulfone having a weight average molecular weight of 20000D with crosslinking linear peptides that contain multiple cysteines (e.g. GCRPQGIWGQDRC) at pH 7.5. The PEG was dissolved in a TEOA buffer (0.3M, pH 8.0) to give a 10% (w/w) solution. The peptide was dissolved in the same buffer. The thiolates that were present reacted with the unsaturated moiety, giving an end crosslinked hydrogel. By incorporating degradation sequences between the two cysteines that are specifically sensitive to either plasmin or collagenase, a synthetic substitute for fibrin and collagen (respectively) can be created. Through the addition of adhesion signals, usually ROD peptides, these gels can serve as a cell infiltration matrix and delivery matrix for bioactive molecules.

Synthetic gels of described above were created with 5 µg of deglycosylated rh-BMP-2 precipitated in the matrix and placed inside the 8mm critical size rat cranial defect. They were explanted after one, three and five weeks. No signs of inflammation or scar tissue were observed. Furthermore, the healing rate was 80% after five weeks, indicating that these synthetic matrices serve as suitable matrices for precipitation of rh-BMP-2 and act as healing matrices.

### In vivo work with collagen matrices

Clinically available adsorbable collagen sponges (Integra Lifesciences) were obtained and cut into the appropriate shape for the rat critical size cranial defect. In order to prepare them for implantation, these sponges were then soaked in a solution containing 5 µg of nonglycosylated BMP-2.

Defects of 8 mm were created in rat crania and a collagen sponge with rh-BMP-2 entrapped was placed inside the defect. The amount of regenerated bone within the defect at both three and five weeks was measured radiographically to determine the efficacy of precipitated rh-BMP-2 in bone regeneration.

When the collagen sponges which contained 5 µg rh-BMP-2, were explanted, there was no indication of an adverse reaction to the implanted material. In every sample, there was no sign of inflammation or scarring at the site of healing or on the dura. A total of seven samples were tested, with three tested at the 3 week timepoint and 4 at the 5 week timepoint. When samples were explanted at three weeks, each of the defects was completely filled with calcified tissue. After five weeks, a similar result was observed where radiographically, 94% of the defect was filled with woven bone. Clearly then, the addition of a nonglycosylated BMP-2 in a collagen matrix provided excellent healing. This demonstrates that retention of BMP-2 within a matrix by utilization of a nonglycosylated form is also functional in collagen sponges.

### Example 6: Healing in the canine Pancarpal Arthrodesis

The components for the gels were prepared such that the final concentration obtained were 8 mg/ml fibrinogen, 2.5 mM Ca⁺⁺, 10 NIH Units/ml of thrombin and 600 µg nonglycosylated rhBMP-2/ml gel. Gelation began after mixing and injection of the components into the fracture site. Gelation time was 30-60 seconds, the contamination of the components with small amounts of blood in the wound did not influence the gelation properties.

Ten consecutive cases of client-owned dogs requiring a carpal panarthrodesis after a trauma were operated at the Small Animal Clinic of the University of Berne. The standard technique of dorsal plating was applied in all dogs. After reaming the joint cartilage, a plate of appropriate size was fixed with screws using the AO-technique. The operation field was than flushed with physiological NaCl-solution and the fibrin/ng-rhBMP-2-solution injected into the bone gaps (10-40 µg ngly-rhBMP-2/ kg body weight). The gelation took 30-60 seconds to complete. The wound was closed routinely using absorbable suture material.

One dog (dog 10) suffered from a bilateral carpal injury after a fall. On both carpi, a panartrodesis was performed by the same blinded surgeon at the same day, and at the end of the procedure, the carpi were randomly selected to receive a spongiosa autotransplant or fibrin/rhBMP-2. While this case could not be included in the statistical analysis due to the bilateral injury, it did provide a direct internal comparison between autograft and fibrin/rhBMP-2.

After the postoperative radiographs, a protective splint was adapted. Limitation of free running and weekly bandage control was recommended for six weeks as was performed in the control group. Standard control radiographs were taken at four, eight, and twelve weeks postoperatively. The dogs were clinically examined at the same time points and their gait was evaluated. The radiographic bony healing was judged using a scoring system by an independent board certified radiologist (GS) and the results compared to a control group of 17 dogs which were operated with the same technique but using a spongiosa autograft.

Scoring system: 0= no mineralized tissue in the joint gap visible, 1= mineralized tissue in the joint gap visible, 2= bony bridging of the joint gap, 3= remodeled bony bridging with absent subchondral plate.

No dog showed local or systhemic signs of adverse drug reactions, and the operation wounds consistently healed uneventfully.

Only minor complications occurred in some patients related to the splint (small pressure wounds) which were managed by changing the bandage and cleaning of the irritated skin.

The mean radiologic healing score was at all time points (4,8,12 weeks) greater in the non-glycosylated (ngly)-rhBMP-2 group than in the spongiosa group ( p _{4 weeks}= 0.0063, p _{8 weeks} = 0.115, p _{12 weeks} = 0.268) (Fig. 5).

At 12 weeks post operative 59% of the spongiosa group reached a score of 2 or greater in all joints (the standard level indicating clinical healing), whereas 87.5% of the ngly-rhBMP-2 group reached that score.

Dog 10 with the bilateral panarthrodesis had a post operative period without complications. The first control radiograph after 4 weeks showed no visible difference in the bony healing of the two arthrodesis (score 1 for all joints). However, after eight weeks the spongiosa treated carpus had no improvement (score 1), whereas the ngly-rhBMP-2 treated carpus improved to a score of 2. After twelve weeks the score for the spongiosa treated leg was 2, and 2.33 for the ngly-rhBMP-2 treated arthrodesis.

The radiographs taken at later time points demonstrated further healing and remodeling of the arthrodesis without forming bone outside the desired area, and no lysis or resorption of the induced bone was visible and no clinical problems developed in the operated animals in a post operative time range up to 14 months.

### Example 7: Healing in the Feline Long Bone Non-Union

The components for the gels were prepared such that the final concentration obtained were 8 mg/ml fibrinogen, 2.5 mM Ca⁺⁺, 10 NIH Units/ml of thrombin and 600 µg nonglycosylated rhBMP-2/ml gel. Gelation was allowed after mixing and injection of the components into the fracture site. Gelation time was 30-60 seconds, the contamination of the components with small amounts of blood in the wound did not influence the gelation properties.

Five consecutive cases of fracture nonunions in client-owned short hair cats, 3 male and 2 female with the mean age of 3.4 years (2 to 10 years), were treated at the Veterinary Teaching Hospital of the University of Berne. Each patient had an atrophic nonunion, that showed no progression in healing for a minimum of three months before they were treated with rhBMP-2. Primary fixation of the fractures were provided by an external fixator in cat #1-4 and im-pinning in cat #5. In cat #1-3 and 5 the primary fixation was unstable, and a plate was applied to gain stability. In the same operation the rhBMP-2 was inserted to the fracture site. In cat #4, the external fixator showed no signs of loosening and the rhBMP-2 was inserted through a stab incision to the fracture gap (table 3). Two months after application of the rhBMP-2 the plate loosened in cat #3 because of a fall. The plate was removed and 300 µg rhBMP-2 in fibrin inserted a second time to the fracture area. A cast was added and after six weeks, an im-pin was inserted to provide stability. In cat #5, a mini plate was placed on the lateral aspect of the metacarpal bone (Mc) 5 to stabilize the fractures together with the intact Mc2. Through a small approach to the fractures of the Mc 3 and 4, fibrin with 300µg rhBMP-2 was injected.

Several control radiographs were taken in all cases during the months following the treatment with fibrin and rhBMP-2.

No cat showed local or systemic signs of adverse drug reactions, the operation wounds healed uneventfully.

**Table 3 Healing in the Feline Non-union**

| Cat | Fracture | Time | Treatment | Result |
|---|---|---|---|---|
| 1 | R/U prox. | 3 month | Plate, 1 x BMP | Healed |
| 2 | R/U dist. | 12 month | Plate, 1x BMP | Healed |
| 3 | Tibia | 12 month | Plate, 1x BMP Cast, 1x BMP im-pin | Healed |
| 4 | Tibia | 4 month | 1x BMP (ExFix) | Healed |
| 5 | Mc 3,4,5 | 5 month | Plate, 1x BMP | Not Healed |

In cat #1, four weeks after treatment new calcified tissue was visible on the radiographs in the fracture area. Four months after the application of the rhBMP-2 the fracture was healed, and the cat showed no lameness.

In cat #2, the fracture gap after the placement of a mini-T-plate was small, and six weeks after the treatment with rh-BMP2 the fracture was bridged with excellent limb function.

Cat #3 suffered a very comminuted tibial fracture, which was stabilized by an external fixateur. The tibia developed an atrophic nonunion with severe bone loss. A 2.7mm-glate was applied after shortening of the fibula to reduce the gap of the tibia. The bone from the fibula was morselised and mixed in the fibrin with the rhBMP-2 to provide living cells to the fracture site. The follow up radiographs showed new bone formation and building up of a new cortex along the whole tibia. After the pull out of the distal screws because of a trauma the plate was removed, and 300 µg rhBMP-2 in fibrin was applied a second time. The bone continued to augment, and six months after the first rhBMP-2 treatment the fracture has healed.

Cat #4 had an open tibial fracture, which was stabilized by an external fixateur. After a mild, transient osteomyelitis the bone of the tibia started to atrophy despite the stable conditions. The fibrin/rhBMP-2 was applied through a stab incision in the fracture gap. After four weeks no bony reaction was visible on the radiographs, but after seven weeks the fracture gap was smaller, and 4 months after the treatment the bone has bridged.

In cat #5, the metatarsal bones 3,4 and 5 severely atrophied after the stabilization of comminuted fractures with im-pinning. The control radiographs revealed no effect of the rhBMP-2 after four and seven weeks. The owner denied further treatment, no longer follow up was possible.

### Example 8 Rentention of non-glycosylated rhPDGF-AB in fibrin and synthetic matrix

This in vitro assay assesses the retention of non-glycosylated rhPDGF-AB in fibrin and a synthetic matrix. PDGF-AB is known to contain a N-glycosylation site on the A chain which is suggested to be used when the protein is expressed by a eukaryotic cell. rhPDGF-AB expressed in *E-Coli,* which is expected to be non-glycosylated, is used in this study and soluble in physiological pH up to 0.2 mg/mL. (Hoppe, J. et al, Biochemistry, 28, 2956-60 (1989); Hoppe, J. et al. Eur J Biochem, 187, 207-14 (1990))

Non-glycosylated PDGF-AB was tested at 2 µg per 50 µL gels. Fibrin gels were polymerised using a modified formulation of Tissucol (Baxter) and the synthetic gel was of 4-armed PEG-acrylate cross-linked with 2-armed PEG-thiol. The gels were washed in buffered saline (PBS 0.01 M, pH 7.4, with 0.1% BSA) and the wash changed after 12 hours. The amount of therapeutic molecules released in the wash was then determined by ELISA.

After 20 wash volumes, 0.7 µg of protein was detected in the wash buffer of the fibrin matrix, which results in a retention of 65% total protein load in the fibrin matrix. In the synthetic matrix, less than 4 ng was released after 20 wash volumes, as no protein was detected within the sensitivity of the assay, suggesting full retention of the protein load. As control against interference of matrix in the assay or degradation of the protein, spiked samples were tested and results showed that significant amount of protein was detectable after 20 wash volumes.

Based on these results, it is shown that the prokaryotic rhPDGF-AB can be retained within matrices, such as fibrin and synthetic PEG-gels, to be used in the promotion of healing applications.

It is understood that the disclosed invention is not limited to the particular methodology, protocols, and reagents described herein. Further, the terminology used herein is for the purpose of describing particular embodiments, and is not intended to limit the scope of the present invention.

Those skilled in the art Will recognize, or be able to ascertain using no more than routine experimentation, many equivalents to the specific embodiments of the invention described herein. Such equivalents are intended to be encompassed by the following claims.

## Claims

1. A system for wound healing comprising at least a first and a second composition separated from each other,
wherein
the first composition comprises a three dimensional network precursor component,
the second composition comprises a further three dimensional network precursor component,
the precursor components, when mixed under conditions that allow polymerization of the precursor components, form a three dimensional fibrin network,
and further wherein
at least one of the first or second composition comprises a biologically active molecule being selected from deglycosylated members of the cystine knot growth factor superfamily.

2. The system according to claim 1 wherein the first composition comprises fibrinogen.

3. The system according to any of the claims 1 to 2 wherein the second compostion comprises thrombin.

4. The system according to any of the claims 1 to 3 wherein at least one of the first or second composition further comprises a calcium source.

5. The system of claim 1 wherein the first composition comprises fibrinogen and thrombin and the second composition comprises a calcium source.

6. The system according to any of the claims 1 to 5 wherein the bioactive molecule is a deglycosylated member of the TGF b superfamily.

7. The system of claim 6 wherein the bioactive molecule is a deglycosylated member of the bone mophogenetic protein.

8. The system of claim 7 wherein the bioactive molecule is deglycosylated rh-BMP2.

9. The system of claim 6 wherein the bioactive molecule is a platelet derived growth factor (PDGF) and in particular PDGF AB.

10. The system according to any of the claims 1 to 9 wherein the conditions under which the components form a three dimensional matrix are physiological conditions in the human or animal body.

11. Use of the system according to any of the claims 1 to 10 for the manufacture of a three dimensional network for use as a matrix for wound healing and tissue regeneration.

12. Use of claim 11 wherein the wound is a bony defect.

13. Use of claim 11, wherein the wound is a cutaneous chronic wound.

14. A composition for wound healing comprising:
a polymeric matrix consisting of fibrin and
a bioactive molecule physically entrapped in the polymeric matrix,
wherein
the bioactive molecule is a deglycosylated member of the cystine knot growth factor superfamily.

15. The composition of claim 14 wherein the bioactive molecule is a deglycosylated member of the TGFb superfamily.

16. The composition of claim 15, wherein the bioactive molecule is a deglycosylated bone morphogenetic protein.

17. The composition of claim 16, wherein the bioactive molecule is deglycosylated rh-BMP-2.

18. The composition of claim 15, wherein the bioactive molecule is a deglycosylated platelet derived growth factor, in particular PDGF AB.

19. A composition of any of the claims 14 to 18, wherein the bioactive molecule is physically entrapped by precipitation within the matrix.

20. A method of forming a matrix which improves wound healing, comprising mixing the first and second composition of the system according to any of the claims 1 to 10.

21. The method according to claim 20 further comprising coupling a second, different bioactive molecule to the composition, wherein the polymeric matrix includes sites with binding affinity for the second bioactive molecule and
wherein the first bioactive molecule has binding affinity for the second bioactive molecule.

22. The method according to claim 20 or 21, wherein the bioactive molecule is precipitated during formation of the matrix.

23. The method according to claim 20 or 21 wherein the bioactive molecule is precipitated before formation of the matrix.

24. A method of decreasing the solubility of a deglycosylated member of the cystine knot growth factor superfamily in fibrin matrices, comprising the step of converting the growth factor to a deglycosylated one.

25. The method according to claim 24, wherein the growth factor is selected from the group consisting of a bone morphogenetic protein and platelet derived growth factor (PDGF).

26. The method of claim 25 wherein the growth factor is rh BMP-2.

27. The method of claim 25 wherein the growth factor is PDGF AB.

28. A method of forming a composition which improves wond healing, comprising precipitating a bioactive molecule within a polymeric fibrin matrix,
wherein the bioactive molecule is a non-glycosylated member of the cystine knot growth factor superfamily.

29. The method of claim 28, wherein the bioactive molecule is a non-glycosylated member of the TGF b superfamily.

30. The method of claim 29, wherein the bioactive molecule is a non-deglycosylated member of a bone morphogenetic protein.

31. The method of claim 30 wherein the non-glycosylated member of a bone morphogenetic protein is rh-BMP-2.

32. The method of any of the claims 28 to 31, further comprising coupling a second, different bioactive molecule to the composition wherein the polymeric matrix includes sites with binding affinity for the second bioactive molecule and wherein the first bioactive molecule has binding affinity for the second bioactive molecule.

33. The method of claim 32, wherein the first bioactive molecule is a bone morphogenetic protein.

34. The method of claim 33, wherein the first bioactive molecule is rh-BMP-2.

35. The method according to any of the claims 32 to 34, wherein the second bioactive molecule is heparin.

36. A device containing the system according to any of the claims 1 to 10.

37. The device of claim 36, wherein the device is a two compartment syringe wherein the first compartment contains the first composition and the second compartment contains the second composition and the two compartments are combined by a two way connector.

38. The device of claim 37 wherein the two compartments are bipartite and separated by an adjustable partition rectangular to the compartment wall.

39. Use of the composition according to any of the claims 14 to 19 for the manufacture of a medicament for healing of wounds.
